(19) Europäisches Patentamt
European Patent Office
Office européen des brevets

(11) Veröffentlichungsnummer: **0 255 691**
**A2**

# (12) EUROPÄISCHE PATENTANMELDUNG

(21) Anmeldenummer: **87110976.5**

(51) Int. Cl.4: **C12P 19/18** , C12N 11/14

(22) Anmeldetag: **29.07.87**

(30) Priorität: **02.08.86 DE 3626213**

(43) Veröffentlichungstag der Anmeldung:
**10.02.88 Patentblatt 88/06**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Thiem, Joachim, Prof. Dr.**
**Asbeckweg 31**
**D-4400 Münster(DE)**
Erfinder: **Treder, Wolfgang**
**Grosse Helkamp 16**
**D-4400 Münster(DE)**
Erfinder: **Keller, Reinhold, Dr**
**Wiesenweg 5**
**D-6232 Bad Soden am Taunus(DE)**
Erfinder: **Schlingmann, Merten, Dr.**
**Schneidhainer Strasse 32a**
**D-6240 Königstein/Taunus(DE)**

(54) **Enzymatische Synthese von Cyclodextrinen mit alpha-Glucosylfluorid als Substrat für Cyclodextrin-alpha (1-4)glucosyl-transferase.**

(57) $\alpha$-Glucosylfluorid läßt sich in Gegenwart von Cyclodextrin-$\alpha$(1→4)glulosyltransferase in hoher Ausbeute zu einem Gemisch von $\alpha$-und $\beta$-Cyclodextrinen umsetzen.

EP 0 255 691 A2

# Enzymatische Synthese von Cyclodextrinen mit $\alpha$-Glucosylfluorid als Substrat für Cyclodextrin-$\alpha$(1→4)-glucosyltransferase

Cyclodextrine sind als Produkte der Umsetzung von Stärke mit Cyclodextrin-$\alpha$(1→4)glucosyltransferase (E.C. 2.4.1.19) aus Bacillus macerans gut bekannt. Die erste aufwendige chemische Synthese von $\alpha$-Cyclodextrin gelang kürzlich T. Ogawa und Y. Takahashi, Carbohydr. Res. 138, C5 (1985). Cyclodextrine sind in der Lage, mit einer großen Anzahl physiologisch wirksamer Substanzen Einschlußkomplexe zu bilden und beanspruchen daher besonderes Interesse in der Pharmaforschung (M.L. Bender und M. Komiyama: "Cyclodextrin Chemistry" Springer Verlag, Berlin (1978). Bisher sind folgende von Cyclodextrin-glucosyltransferase (CGT) katalysierte Reaktionen bekannt [H. Bender, Carbohydr. Res. 78, 133 (1980); K. Wallenfels et al., Carbohydr. Res. 61, 359 (1978); H. Bender, Carbohydr. Res. 78, 147 (1980)]:

1. Cyclisierung: 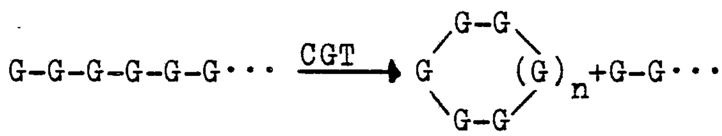


$\alpha$-, $\beta$-, $\gamma$-Cyclodextrin (n=1,2,3)

2. Kupplung: 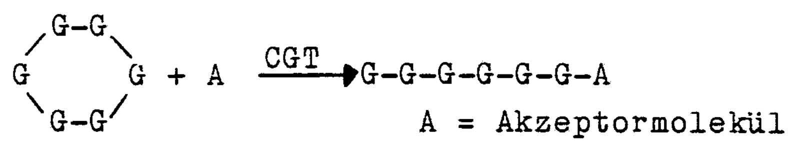


3. Disproportionierung: $G_m + G_n \xrightarrow{CGT} G_{m+x} + G_{n-x}$

In einigen Studien konnte gezeigt werden, daß bestimmte Enzyme in der Kohlenhydratchemie Glycosylfluoride als Substrate für ihre Reaktion akzeptieren [P.J. Card und W.D. Hitz, J. Am. Chem. Soc. 106, 5348 (1984); D.G. Drueckhammer und C.-H. Wong. J. Org. Chem. 50, 5912 (1985); A.M. Gold und M.P. Osber, Biochem. Biophys. Res. Commun. 42, 469 (1971)].

Es wurden überraschend gefunden, daß das Enzym Cyclodextrin-glucosyltransferase {(1→4)-$\alpha$-D-glucan: [(1→4)-$\alpha$-D-glucopyranosyl]transferase (cyclizing), E.C. 2.4.1.19, CGT} fähig ist, $\alpha$-Glucopyranosylfluorid zu einem Gemisch aus Cyclodextrinen und Maltooligomeren umzusetzen:

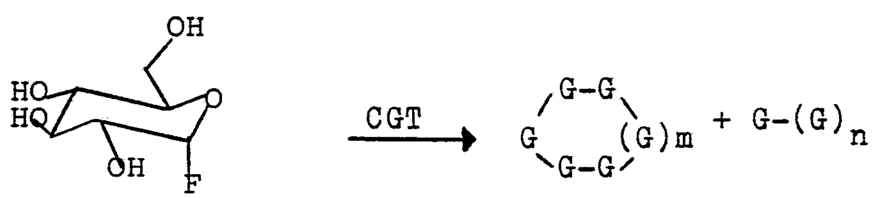


(n = 0 bis 8)

(m = 1 oder 2)

Die Erfindung betrifft somit ein Verfahren zur Herstellung von $\alpha$-und $\beta$-Cyclodextrinen, das dadurch gekennzeichnet ist, daß $\alpha$-Glucopyranosylfluorid mit Cyclodextrin-$\alpha$-(1→4)glucosyltransferase umgesetzt wird.

Im folgenden wird die Erfindung im einzelnen erläutert bzw. in den Patentansprüchen definiert.

Cyclodextrin-$\alpha$-(1→4)glucosyltransferase akzeptiert überraschend $\alpha$-Glucopyranosylfluoride als Substrat. Das Enzym kann sowohl in freier Form als auch in immobilisierter Form eingesetzt werden. Wegen der besseren Raum/Zeit Ausbeute ist die immobilisierte Form bevorzugt. Zu diesem Zweck wird das Enzym im allgemeinen adsorptiv oder kovalent mit oder ohne Spacer an einen Träger gebunden. Geeignet sind organische Träger, wie beispielsweise Polyacrylnitril, oder anorganische Träger, wie z.B. Kieselgel. Besonders bevorzugt ist die kovalente Bindung des Enzyms an den Träger über einen Spacer, insbesondere Glutardialdehyd.

Die Umsetzung des $\alpha$-Glucosylfluorids erfolgt in wäßriger Lösung oder in wäßrig/organischen Gemischen, wobei das Verhältnis von wäßriger Lösung zu organischem Lösungsmittel im Bereich 2:1 bis 1:2, bevorzugt bei 1:1 liegt. Geeignete Lösungsmittelgemische sind beispielsweise Wasser/($C_1$-$C_4$) Alkanole, Wasser/Acetonitril oder Wasser/Aceton. Die Reaktion erfolgt im allgemeinen bei Temperaturen von 20 bis 60 °C, insbesondere bei 40 bis 50 °C und bei einem pH-Wert von 5 bis 8, bevorzugt bei pH 6. Wegen des freiwerdenden Halogenwasserstoffs ist es wichtig, den pH-Wert während der Reaktion zu kontrollieren und entweder mit Hilfe eines Puffersystems, beispielsweise Acetatpuffer, oder durch Zugabe einer Lauge, wie zum Beispiel Natronlauge, in dem gewünschten Bereich zu halten bzw. auf einen bestimmten Wert zu regulieren. Die Reaktionszeit ist abhängig von der für die Reaktion gewählte Temperatur. Arbeitet man im bevorzugten Temperaturbereich, so ist eine Reaktionszeit von 10 bis 60 Minuten zu erwarten.

In den folgenden Beispielen wird die Erfindung weiter im Detail erläutert.

## Beispiele

### 1. Immobilisierung des Enzyms:

Cyclodextrin-$\alpha$(1→4)glucosyltransferase (500 mg = ~ 9000 Units) werden in 10 ml eines 0,05 M Natriumacetatpuffers (pH 6,0) gelöst und bei 20 °C 4 h mit 5 g eines mit Glutardialdehyd funktionalisierten Kieselgelträgers (Grace 332 250 A) geschüttelt [H.H. Weetall, Meth. Enzymol. 44, 134 (1976)]. Anschließend wird das Gel mit 100 ml bidest. Wasser, 200 ml 1 M NaCl und wiederum 200 ml bidest. Wasser gewaschen. Das immobilisierte Enzym ist nun für die Reaktion direkt einsetzbar. Das Gel kann länger als vier Wochen ohne Aktivitätsverlust bei 4 °C aufbewahrt werden.

### 2. Synthese der Cyclodextrine und Maltooligomeren:

Man löst 1,0 g $\alpha$-Glucosylfluorid (5,5 mmol) in 25 ml Natriumacetatpuffer, pH 6,0, und setzt das immobilisierte Enzym zu (1 ml Gel, ~ 1000 Units). Die Reaktion erfolgt bei 45 °C unter leichtem Schütteln, wobei der pH-Wert durch automatische Titration mit 0,5 M NaOH konstant gehalten wird. Nach 20 Minuten ist die Umsetzung beendet, und das Gel kann abfiltriert werden. Zur Reaktionsverfolgung hat sich die DC auf Kieselgelfolien (Propanol/Ethanol/Wasser = 5:3:2; v:v:v) bewährt [K. Koizumi et al.; J. Chromatogr. 321, 145 (1985)].

Ausbeuten: $\alpha$-Cyclodextrin: 300 mg (30 %),
$\beta$-Cyclodextrin: 380 mg (38 %);
Maltooligomere (Glucose bis Maltononaose): 320 mg (32 %).

$R_F$-Werte: $\alpha$-Glc-F: 0,78; Glc: 0,68; $\alpha$-Cyclodextrin: 0,57, $\beta$-Cyclodextrin: 0,54; [$\gamma$-Cyclodextrin: 0,51].

### 3. Analytische Methoden:

Das Reaktionsgemisch läßt sich mit einer RP-18 HPLC-Säule (0,8 x 50 cm, 7 µm, Merck, Darmstadt,) auftrennen [G.D. McGinnis et al., J. Carbohydr. Chem. 5, 83 (1986)]. Bei einer Elutionsgeschwindigkeit von 3 ml/min mit Wasser als Elutionsmittel können zunächst ausschließlich die linearen Maltooligomeren vom Mono-bis zum Nonasaccharid erhalten werden. Dabei bleiben die Cyclodextrine wegen starker Wechselwirkungen auf der Säule. Eluiert man dagegen mit Wasser/Methanol (9:1; v:v) so werden die linearen Maltooligomeren als ungetrennte Fraktion vor den nun sehr gut zu trennenden $\alpha$- und $\beta$-Cyclodextrinen eluiert.

## Ansprüche

1. Verfahren zur Herstellung von α-und β-Cyclodextrinen, das dadurch gekennzeichnet ist, daß α-Glucopyranolsylfluorid mit Cyclodextrin-α-(1→4)glucosyltransferase umgesetzt wird.

2. Verfahren nach Anspruch 1, dadurch gekennzeichnet, daß Cyclodextrin-α(1→4)glucosyltransferase in immobilisierter Form eingesetzt wird.

3. Verfahren nach Anspruch 2, dadurch gekennzeichnet, daß das Enzym an einen anorganischen Träger gebunden eingesetzt wird.

4. Verfahren nach Anspruch 3, dadurch gekennzeichnet, daß das Enzym an Kieselgel gebunden eingesetzt wird.

5. Verfahren nach einem oder mehreren der Ansprüche 1 bis 4, dadurch gekennzeichnet, daß die Reaktion bei Temperaturen von 20 bis 60 °C und einem pH-Wert von 5 bis 8 durchgeführt wird.

6. Verfahren nach Anspruch 5, dadurch gekennzeichnet, daß die Reaktion bei einer Temperatur von 40 bis 50 °C und einem pH-Wert von 6 durchgeführt wird.